# EUROPEAN PATENT SPECIFICATION

(11) **EP 3 902 483 B1**
(45) Date of publication and mention of the grant of the patent: **07.08.2024**
(21) Application number: 19839670.7
(22) Date of filing: 17.12.2019
(51) Int. Cl.: A61B 17/062, A61B 17/04, A61B 17/14, A61B 17/29, A61B 17/11

(54) **SURGICAL NEEDLE GUIDING ROD AND KIT**
CHIRURGISCHE NADELFÜHRUNGSSTANGE UND BAUSATZ
TIGE DE GUIDAGE D'AIGUILLE CHIRURGICALE ET TROUSSE

(30) Priority: 27.12.2018 PT 2018115233
(43) Date of publication of application: 03.11.2021
(73) Proprietor: Pacheco dos Santos Dias, José António, 1250-068 Lisboa (PT)
(72) Inventor: Pacheco dos Santos Dias, José António, 1250-068 Lisboa (PT)
(74) Representative: do Nascimento Gomes, Rui
(86) International application number: PCT/IB2019/060932
(87) International publication number: WO 2020/136504

(56) References cited:
- EP-A1- 1 623 734
- CN-U- 206 285 304
- US-A- 4 911 164
- US-A- 5 342 374
- US-A- 5 480 407
- US-A1- 2006 264 985
- US-A1- 2007 270 888
- US-A1- 2008 140 098
- US-A1- 2014 200 520

## Description

### FIELD OF THE INVENTION

The present invention is enclosed in the area of medical devices, in particular for prostate and urethral surgery, namely for providing anastomosis between the bladder and the urethra of a patient.

### PRIOR ART

Prostate cancer is the most common cancer in men in developed countries. It is the second leading cause of cancer death in men in these countries.

Presently, one of the most frequently performed treatments for the treatment of this type of cancer is surgery, called radical prostatectomy. It can be done openly, laparoscopically or robotically. The tendency to perform this surgery through one of these last two approaches (laparoscopic and robotic) is increasing, given the lower aggressiveness, less invasiveness, lower morbidity and faster recovery of patients.

However, when performed by the latter methodologies and despite the significant advantages to the patient, surgery is technically more demanding for surgeons. One of the technically most difficult steps of prostate cancer surgery is the anastomosis (junction, union) of the bladder with the urethra (called vesico-urethral anastomosis).

It is a very delicate and difficult step, which is performed at the end of the intervention - which means that sometimes it is done after 2 or 3 hours of surgery -, when the surgeons are already more tired, increasing the difficulty of accomplishing some of the gestures needed to complete this step.

Devices known in the art are unsuitable for such purpose. In particular, it is the case of accessories designed for other purposes which are used by urologists to somehow attempt to make this step easier: they are metallic accessories, similar to urethral dilators, with a circular end opening.

This opening is used to facilitate insertion of the tip of the needle into the urethral lumen. However, it does not allow the gesture of insertion of the needle tip through the urethra and consequent rotation of the needle (necessary for the correct and adequate accomplishment of this step) to be done swiftly, safely and effectively, guaranteeing to involve the necessary and sufficient extension of the urethra to ensure a union to the bladder with the necessary resistance to the subsequent function of these organs, often causing a union between the urethra and the bladder that does not become tight, which tears the very delicate urethral tissue and sometimes contributes to aggravate the problem of incontinence that often arises after this type of surgery.

It is the case of the object described in patent application US 2897820.

US patent no. US 5,480,407 describes a suturing instrument for impeding the flow of blood around a suturing site, which comprises a rigid shaft, the instrument defines a rotational suture guiding device which is solid and comprises a tubular section and a suture guide. A point of entry of needle is provided, connected to a slit. The instrument thus comprises a distal end to be inserted into a patient during a suturing procedure.

US patent no US 5,342,374 describes a similar surgical needle-guiding device, which is also based on a tubular shaft with opposite ends, one end distal and another end proximal in relation to a user. The device is also intended to guide and control a suture needle.

US patent no. US 4,911,165 describes yet another similar surgical needle-guiding device, with an elongated body with a straight portion ending in an end portion which has one or more grooves extending from a tip to the end portion.

US patent application no. US 2007/0270888 discloses a catheter for use in the suturing of so-called conduit shaped tissues within a subject's body and which, as previous documents, includes a grooved tip member in a distal end which allows for the tip of a suture needle to be received and guided.]

Thus, the present invention provides a solution to the described issues found in prior art, enhancing the anastomosis operation.

### SUMMARY OF THE INVENTION

It is an object of the present invention a surgical needle guiding rod (1) for providing anastomosis between the bladder and the urethra of a patient as provided in claim 1, such rod (1) thus being solid and comprising a proximal portion (2) and a distal portion (3), a first opening (11) and a second opening (12) being formed on the surface of the distal portion (3) thereby forming a channel (4) through the solid rod (1) between the two openings, the channel (4) being such that it allows a surgical needle to pass by entering the one of the two openings and exiting the other opening. By having a channel (4) formed between the two openings, in the distal portion (3), the rod (1) of the present invention provides for suturing of urethral-bladder tissues and thereby facilitates the anastomosis procedure. Thus, a surgeon may position the rod (1) of the present invention as desired and the configuration of openings allows that a surgical needle inserted in the first opening (11) will be directed into the second opening (12), which would be positioned so to be adjacent to the tissue which the surgeon wishes to suture.

The rod has a curved cross-section, preferably circular, the first opening (11) being formed in the tip of the rod (9) an in a substantially concentric fashion with the axis of the rod (1), and the second opening (12) being formed laterally on the surface of the rod (1). Thus, a curved needle may easily be inserted in the first opening (11), provided in the tip of the rod (9), and exit the rod (1) in the second opening (12), provided on the side. Thus, the device of the present invention - resorting to a simple construction - provides for the guiding of a surgical needle from the entrance of the urethra [where the first opening (11) will be positioned] to its lateral tissue [where the second opening (12) is].

The first opening (11) is wider than the second opening (12), the channel (4) forming a substantially conical shape between the two openings. Therefore, the first opening (11), being wider, provides an easiness of insertion of the needle, and the corresponding shape of the channel (4) (substantially conical) and the second opening (12) (lesser width), allows for the needle to be directed into the second opening (12), to the correct position of the urethral tissue, thus enhancing the anastomosis operation.

In yet another inventive aspect of the rod (1) of the present invention, it further comprises adhering/fixation means (6) provided in the distal portion (3) and actuation means in the proximal portion (2), the adhering/fixation means (6) being such that, when applied in the urethra of a patient and under an actuation action in the actuation means, provide adherence to the urethra of a patient allowing the rod (1) to push and fix the urethra and perineum. Such adhering and actuation means may take several different forms, all providing for i) pushing of the perineum and urethra into a desired position and ii) maintaining a correct position of the surgical rod (1) in an anastomosis operation, as defined by a surgeon. After positioning and fixation by operation of the actuation means, suturing as previously described may be performed (additionally) much more easily and without the risk that changes occur in the positioning of the urethra with respect to the distal portion (3) of the rod (1).

It is also an object of the present invention a surgical suture kit according to claim 15 comprising the rod (1) of any of the preceding claims and a surgical needle, such surgical needle having a curvature, the curvature preferably being of 3/8 circle, 1/2 circle or 5/8 circle, more preferably with a chord length of 16 to 30 mm.

### DESCRIPTION OF FIGURES

Figure 1 - frontal view of a representation of an embodiment of the guiding rod (1) of the present invention, specifically of a part of the distal portion (3), in which the second opening (12) and the slot (5) are viewable. The channel (4) between the second opening (12) and the first opening (11) is represented in streak. In such case, the first opening (11) is wider than the second opening (12), providing a conical channel (4), and the slot (5) allows for an easier removal of a needle.
Figure 2 - section view of a representation of an embodiment of the guiding rod (1) of the present invention, specifically of a part of the distal portion (3), in which the second opening (12), the first opening (11) and the channel (4) are viewable. The channel (4) has a conical and curved shape.
Figure 3 - side view of a representation of an embodiment of the guiding rod (1) of the present invention, containing both the proximal portion (2) and the distal portion (3), such rod (1) having a distal portion (3) according to the embodiments of figures 1 and 2. The rod (1) further comprises a proximal button (7) which activates the rotation of the distal tip section (8).

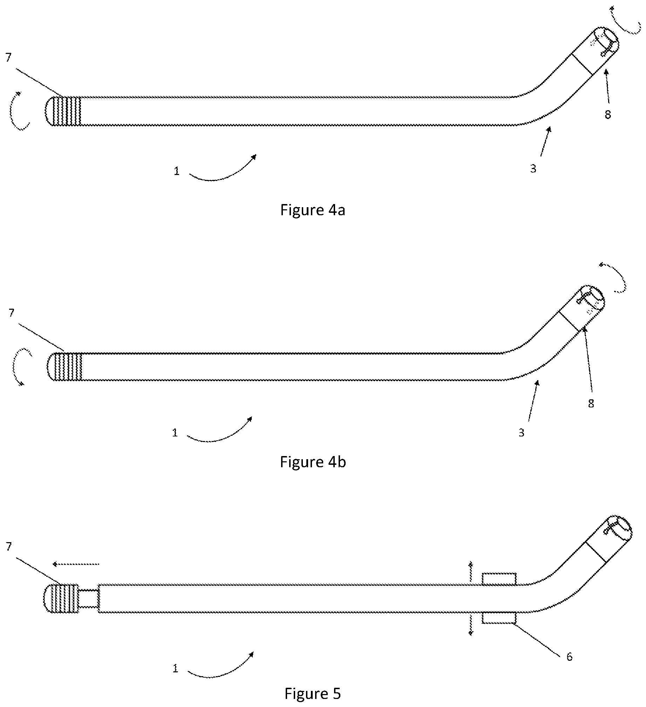

Figures 4a and 4b - representation of the actuation of the embodiment of figure 3, providing the rotation of the distal tip section (8).
Figure 5 - representation of a second actuation of the embodiment of figure 3, providing wherein actuation means, which in such case consist of the proximal button (7), provide inflation of a balloon or protrusion from the surface of bulking parts [the adhering/fixation means (6)].

### DETAILED DESCRIPTION

The more general and advantageous configurations of the present invention are described in the Summary of the invention. Such configurations are detailed below in accordance with other advantageous and/or preferred aspects of the present invention.

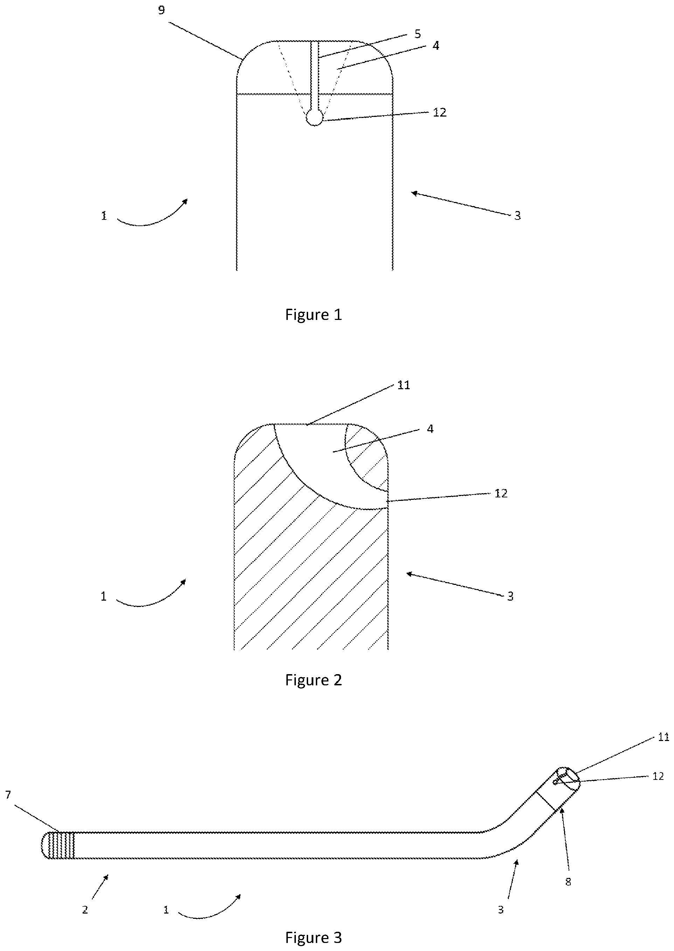

In preferred embodiment of the rod (1) of the present invention, the channel (4) is curved. The curvature provides an enhanced guidance of a surgical needle. An implementation of such embodiment is shown in Fig. 2.

In another preferred embodiment of the rod (1) of the present invention, combinable with any above described, it has a straight section and a curved section, the curved portion comprising the distal section and the straight portion comprising the proximal section. Such curved section provides for easiness of insertion of the device, as well as it enhances the positioning of the first (11) and second openings (12), for subsequent suturing.

In another inventive aspect of the rod (1) of the present invention, a slot (5) is formed on the surface of the rod (1) between the two openings and the channel (4), the width of the slot (5) being less than the width of the second opening (12). A sowing string is connected to the needle that passes through the channel (4), and the slot (5) thereby allows for an eased removal of the needle.

Preferably, the width of the first opening (11) is of 3-6 mm, and the width of the second opening (12) is of 2-4 mm. Preferably, the width of its cross section is of 7-9 mm, preferably 8 mm.

In another inventive aspect of the rod (1) of the present invention, it further comprises a proximal button (7) provided in the tip of the proximal portion (2) and a transmission mechanism, and a distal tip section (8) comprising the two openings is rotatable, where the proximal button (7) and the transmission mechanism are so arranged that, under rotation of the proximal button (7), the transmission mechanism provides rotation of the distal tip section (8) and, consequently, of the two openings and the respective channel (4) formed therein. Such embodiment provides for an easiness of operation, which does not require the surgeon to rotate the whole rod (1) to pass from a position of the second opening (12) - from which the needle exits the channel (4) - to a second position, to continue the suturing operation. With such transmission mechanism and proximal button (7), the distal tip section (8) - a section defined by comprising the two openings (first and second) - also rotates and therefore allows to position the second opening (12) adjacently to another section of the urethra.

In an embodiment of the rod (1) of the present invention, it is made of a metallic material suitable for human surgical application, preferably stainless steel or titanium. Such material is thus reusable.

In another - alternative - embodiment of the rod (1) of the present invention, it is made of a polymer material suitable for human surgical application, preferably polyether block amide, polytetramethylene glycol, an elastomer (a thermoplastic elastomer such as thermoplastic polyurethane, polyester elastomers, silicones, or polyethylene). Such material is thus not reusable.

In another embodiment of the rod (1) of the present invention, the proximal portion (2) comprises gripping means, preferably provided in the tip of the proximal portion (2).

Reference is made to the embodiment of the rod (1) which further comprises adhering/fixation means (6) provided in the distal portion (3) and actuation means in the proximal portion (2), the adhering/fixation means (6) being such that, when applied in the urethra of a patient and under an actuation action in the actuation means, provide adherence to the urethra of a patient. In a specific embodiment, the adhering/fixation means (6) consist of a balloon or a ring, arranged over the surface of the rod (1) and the actuation means of an element configured to provide inflation of such balloon, whereby an inflation channel (4) is formed inside the rod (1) between such inflation element and the inner side of the balloon.

Preferably, said inflation element consists of i) an inflation opening in the tip of the proximal portion (2) and connected to the inflation channel (4) and ii) an inflation element, preferably a syringe, connectable to said inflation opening whereby, under actuation of the inflation element while connected to the inflation opening, the balloon fills with a saline solution.

In another - alternative - specific embodiment, the adhering/fixation means (6) consist of at least three hinged arms or bulking parts which in an unarmed position are arranged at the surface of the rod (1) and, in an armed position reached under said actuation, protrude from the surface, the actuation means consisting of an element configured to provide actuation of said hinged arms.

Preferably, said element configured to provide actuation of said hinged arms consists of a button and a transmission mechanism connected between the button and the hinged arms and displaced on the inside of the rod (1) whereby, under actuation of the button, the transmission mechanism provides said protrusion of the hinged arms from the surface.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

## Claims

1. A surgical needle guiding rod (1) for providing anastomosis between the bladder and the urethra of a patient which is solid and comprises a proximal portion (2) and a distal portion (3), a first opening (11) and a second opening (12); said openings being formed on the surface of the distal portion (3) thereby forming a channel (4) through the solid rod (1) between the two openings, the channel (4) being such that it allows a surgical needle to pass by entering one of the two openings and exiting the other opening,
wherein the rod has a curved cross-section, preferably circular, the second opening (12) being formed laterally on the surface of the rod (1),
the rod being **characterised in that**
the first opening (11) is formed in the tip of the rod (9) in a substantially concentric fashion with the axis of the rod (1), and the first opening (11) is wider than the second opening (12), the channel (4) forming a substantially conical shape between the two openings.

2. A rod (1) according to the previous claim wherein the channel (4) is curved.

3. A rod (1) according to any of the preceding claims wherein it has a straight section and a curved section, the curved portion comprising the distal section and the straight portion comprising the proximal section.

4. A rod (1) according to any of the preceding claims, wherein a slot (5) is formed on the surface of the rod (1) between the two openings and the channel (4), the width of the slot (5) being less than the width of the second opening (12).

5. A rod (1) according to any of the preceding claims wherein the width of the first opening (11) is of 1-4 mm, and the width of the second opening (12) is of 2-8 mm.

6. A rod (1) according to any of the preceding claims, wherein the width of its cross section is of 4-9 mm, preferably 8 mm.

7. A rod (1) according to any of the preceding claims wherein it further comprises adhering/fixation means (6) provided in the distal portion (3) and actuation means in the proximal portion (2), the adhering/fixation means (6) being such that, when applied in the urethra of a patient and under an actuation action in the actuation means, provide adherence to the urethra of a patient thereby allowing the rod (1) to push and fix the urethra and perineum.

8. A rod according to the previous claim wherein the adhering/fixation means (6) consist of a balloon arranged over the surface of the rod (1) and the actuation means of an element configured to provide inflation of such balloon, whereby an inflation channel (4) is formed inside the rod (1) between such inflation element and the inner side of the balloon and, preferably:
• said inflation element consists of i) an inflation opening in the tip of the proximal portion (2) and connected to the inflation channel (4) and ii) an inflation element, preferably a syringe, connectable to said inflation opening whereby, under actuation of the inflation element while connected to the inflation opening, the balloon fills with a saline solution.

9. A rod (1) according to any of the claims 1-6 wherein it further comprises adhering/fixation means (6) provided in the distal portion (3) and actuation means in the proximal portion (2), the adhering/fixation means (6) being such that, when applied in the urethra of a patient and under an actuation action in the actuation means, provide adherence to the urethra of a patient.

10. A rod according to the previous claim wherein the adhering/fixation means (6) consist of at least three hinged arms or bulking parts which in an unarmed position are arranged at the surface of the rod (1) and, in an armed position, reached under said actuation, protrude from the surface, and the actuation means consisting of an element configured to provide actuation of said hinged arms and, preferably:
• said element configured to provide actuation of said hinged arms or bulking parts consists of a button and a transmission mechanism connected between the button and the hinged arms or bulking parts and displaced on the inside of the rod (1) whereby, under actuation of the button, the transmission mechanism provides said protrusion of the hinged arms from the surface.

11. A rod (1) according to any of the claims 1-8 wherein it further comprises a proximal button (7) provided in the tip of the proximal portion (2) and a transmission mechanism, and a distal tip section (8) comprising the two openings is rotatable, where the proximal button (7) and the transmission mechanism are so arranged that, under rotation of the proximal button (7), the transmission mechanism provides rotation of the distal tip section (8) and, consequently, of the two openings and the respective channel (4) formed therein.

12. A rod (1) according to any of the preceding claims wherein it is made of a metallic material suitable for human surgical application, preferably stainless steel or titanium.

13. A rod (1) according to any of the claims 1-11 wherein it is made of a polymeric material, preferably polyether block amide, polytetramethylene glycol, an elastomer, or polyethylene.

14. A rod (1) according to any of the preceding claims wherein the proximal portion (2) comprises gripping means, preferably provided in the tip of the proximal portion (2).

15. A surgical suture kit comprising the rod (1) of any of the preceding claims and a surgical needle, such surgical needle having a curvature.

## Patentansprüche

1. Chirurgischer Nadelführungsstab (1) zum Herstellen einer Anastomose zwischen der Blase und der Harnröhre eines Patienten, der massiv ist und einen proximalen Abschnitt (2) und einen distalen Abschnitt (3), eine erste Öffnung (11) und eine zweite Öffnung (12) umfasst; wobei die Öffnungen auf der Oberfläche des distalen Abschnitts (3) ausgebildet sind, wodurch ein Kanal (4) durch den massiven Stab (1) zwischen den beiden Öffnungen gebildet wird, wobei der Kanal (4) so beschaffen ist, dass er den Durchgang einer chirurgischen Nadel ermöglicht, indem er in eine der beiden Öffnungen eintritt und aus der anderen Öffnung austritt,
wobei der Stab einen gekrümmten, vorzugsweise kreisförmigen Querschnitt aufweist und die zweite Öffnung (12) seitlich auf der Oberfläche des Stabes (1) ausgebildet ist,
der Stab **dadurch gekennzeichnet ist, dass**
die erste Öffnung (11) ist in der Spitze des Stabes (9) im Wesentlichen konzentrisch zur Achse des Stabes (1) ausgebildet, und die erste Öffnung (11) breiter ist als die zweite Öffnung (12), wobei der Kanal (4) zwischen den beiden Öffnungen eine im Wesentlichen konische Form bildet.

2. Stab (1) nach dem vorhergehenden Anspruch, wobei der Kanal (4) gekrümmt ist.

3. Stab (1) nach einem der vorhergehenden Ansprüche, wobei er einen geraden Abschnitt und einen gekrümmten Abschnitt aufweist, wobei der gekrümmte Abschnitt den distalen Abschnitt und der gerade Abschnitt den proximalen Abschnitt umfasst.

4. Stab (1) nach einem der vorhergehenden Ansprüche, wobei auf der Oberfläche des Stabes (1) zwischen den beiden Öffnungen und dem Kanal (4) ein Schlitz (5) ausgebildet ist, wobei die Breite des Schlitzes (5) geringer ist als die Breite der zweiten Öffnung (12).

5. Stab (1) nach einem der vorhergehenden Ansprüche, wobei die Breite der ersten Öffnung (11) 1-4 mm und die Breite der zweiten Öffnung (12) 2-8 mm beträgt.

6. Stab (1) nach einem der vorhergehenden Ansprüche, wobei die Breite seines Querschnitts 4-9 mm, vorzugsweise 8 mm, beträgt.

7. Stab (1) nach einem der vorangehenden Ansprüche, wobei er ferner eine Haft-/Fixierungsmittel (6), die im distalen Abschnitt (3) vorgesehen ist, und eine Betätigungsmittel im proximalen Abschnitt (2) umfasst, wobei die Haft-/Fixierungsmittel (6) so beschaffen ist, dass sie, wenn sie in der Harnröhre eines Patienten und unter einer Betätigungswirkung in der Betätigungsmittel angebracht wird, für eine Haftung an der Harnröhre eines Patienten sorgt, wodurch der Stab (1) die Harnröhre und das Perineum drücken und fixieren kann.

8. Stab nach dem vorhergehenden Anspruch, wobei die Haft-/Fixierungsmittel (6) aus einem Ballon bestehen, der über der Oberfläche des Stabes (1) angeordnet ist, und die Betätigungsmittel aus einem Element bestehen, das so konfiguriert ist, dass es für das Aufblasen eines solchen Ballons sorgt, wobei ein Aufblaskanal (4) im Inneren des Stabes (1) zwischen einem solchen Aufblaselement und der Innenseite des Ballons ausgebildet ist, und vorzugsweise:
• das Aufblaselement besteht aus i) einer Aufblasöffnung in der Spitze des proximalen Abschnitts (2), die mit dem Aufblaskanal (4) verbunden ist, und ii) einem Aufblaselement, vorzugsweise einer Spritze, das mit der Aufblasöffnung verbunden werden kann, wodurch sich der Ballon unter Betätigung des Aufblaselements, während es mit der Aufblasöffnung verbunden ist, mit einer Kochsalzlösung füllt.

9. Stab (1) nach einem der Ansprüche 1-6, wobei er ferner eine Haft-/Fixierungsmittel (6), die im distalen Abschnitt (3) vorgesehen ist, und eine Betätigungsmittel im proximalen Abschnitt (2) umfasst, wobei die Haft-/Fixierungsmittel (6) so beschaffen ist, dass sie, wenn sie in der Harnröhre eines Patienten und unter einer Betätigungswirkung in der Betätigungsmittel angebracht wird, eine Haftung an der Harnröhre eines Patienten bewirkt.

10. Stab nach dem vorhergehenden Anspruch, wobei die Haft-/Fixierungsmittel (6) aus mindestens drei Gelenkarmen oder voluminösen Teilen bestehen, die in einer nicht aktivierten Position an der Oberfläche des Stabes (1) angeordnet sind und in einer aktivierten Position, die unter der genannten Betätigung erreicht wird, von der Oberfläche hervorstehen, und die Betätigungsmittel aus einem Element bestehen, das zur Betätigung der genannten Gelenkarme konfiguriert ist, und vorzugsweise:
• das genannte Element, das zur Betätigung der genannten Gelenkarme oder voluminösen Teile konfiguriert ist, aus einem Knopf und einem Übertragungsmechanismus besteht, der zwischen dem Knopf und den Gelenkarmen oder voluminösen Teilen verbunden ist und auf der Innenseite des Stabes (1) angeordnet ist, wobei unter Betätigung des Knopfs der Übertragungsmechanismus für das genannte Hervorstehen der Gelenkarme von der Oberfläche sorgt.

11. Stab (1) nach einem der Ansprüche 1-8, wobei er ferner einen proximalen Knopf (7), der in der Spitze des proximalen Abschnitts (2) vorgesehen ist, und einen Übertragungsmechanismus umfasst, und ein distaler Spitzenabschnitt (8), der die beiden Öffnungen umfasst, drehbar ist, wobei der proximale Knopf (7) und der Übertragungsmechanismus so angeordnet sind, dass bei Drehung des proximalen Knopfes (7) der Übertragungsmechanismus eine Drehung des distalen Spitzenabschnitts (8) und folglich der beiden Öffnungen und des jeweiligen darin gebildeten Kanals (4) bewirkt.

12. Stab (1) nach einem der vorhergehenden Ansprüche, wobei er aus einem für die Anwendung in der Humanchirurgie geeigneten metallischen Material, vorzugsweise rostfreiem Stahl oder Titan, hergestellt ist.

13. Stab (1) nach einem der Ansprüche 1 bis 11, wobei er aus einem polymeren Material, vorzugsweise Polyetherblockamid, Polytetramethylenglykol, einem Elastomer oder Polyethylen, hergestellt ist.

14. Stab (1) nach einem der vorhergehenden Ansprüche, wobei der proximale Abschnitt (2) Greifmittel umfasst, die vorzugsweise an der Spitze des proximalen Abschnitts (2) vorgesehen sind.

15. Chirurgisches Nahtmaterial-kit mit dem Stab (1) nach einem der vorhergehenden Ansprüche und einer chirurgischen Nadel, wobei die chirurgische Nadel eine Krümmung aufweist.

## Revendications

1. Tige de guidage d'aiguille chirurgicale (1) pour réaliser une anastomose entre la vessie et l'urètre d'un patient, laquelle est solide et comprend une partie proximale (2) et une partie distale (3), une première ouverture (11) et une deuxième ouverture (12); lesdites ouvertures sont formées sur la surface de la partie distale (3) formant ainsi un canal (4) à travers la tige solide (1) entre les deux ouvertures, le canal (4) étant de façon à permettre à une aiguille chirurgicale de passer en entrant dans l'une des deux ouvertures et en sortant par l'autre ouverture,
où la tige a une section transversale incurvée, de préférence circulaire, la deuxième ouverture (12) étant formée latéralement sur la surface de la tige (1),
la tige étant **caractérisée par le fait que**
la première ouverture (11) est formée à l'extrémité de la tige (9) de façon substantiellement concentrique à l'axe de la tige (1), et la première ouverture (11) est plus large que la deuxième ouverture (12), le canal (4) constituant une forme substantiellement conique entre les deux ouvertures.

2. Tige (1) selon la revendication précédente, dans laquelle le canal (4) est incurvé.

3. Tige (1) selon l'une quelconque des revendications précédentes, laquelle présente une section droite et une section incurvée, la partie incurvée comprenant la section distale et la partie droite comprenant la section proximale.

4. Tige (1) selon l'une quelconque des revendications précédentes, dans laquelle une fente (5) est formée sur la surface de la tige (1) entre les deux ouvertures et le canal (4), la largeur de la fente (5) étant inférieure à la largeur de la deuxième ouverture (12).

5. Tige (1) selon l'une quelconque des revendications précédentes, dans laquelle la largeur de la première ouverture (11) est de 1 à 4 mm, et la largeur de la deuxième ouverture (12) est de 2 à 8 mm.

6. Tige (1) selon l'une quelconque des revendications précédentes, dans laquelle la largeur de sa section transversale est de 4 à 9 mm, de préférence 8 mm.

7. Tige (1) selon l'une quelconque des revendications précédentes, laquelle comprend en outre des moyens d'adhérence/fixation (6) prévus dans la partie distale (3) et des moyens d'actionnement dans la partie proximale (2), les moyens d'adhérence/fixation (6) étant de façon que, lorsqu'ils sont appliqués dans l'urètre d'un patient et sous une action d'actionnement aux moyens d'actionnement, ils assurent l'adhérence à l'urètre d'un patient, permettant ainsi à la tige (1) de pousser et de fixer l'urètre et le périnée.

8. Tige selon la revendication précédente, dans laquelle les moyens d'adhérence/fixation (6) consistent en un ballonnet disposé sur la surface de la tige (1) et les moyens d'actionnement en un élément configuré pour gonfler ce ballonnet, de sort qu'un canal de gonflage (4) est formé à l'intérieur de la tige (1) entre cet élément de gonflage et la face interne du ballonnet et, de préférence :
• ledit élément de gonflage consiste en i) une ouverture de gonflage dans l'extrémité de la partie proximale (2) et reliée au canal de gonflage (4) et ii) un élément de gonflage, de préférence une seringue, pouvant être relié à ladite ouverture de gonflage de sorte que, sous l'action de l'élément de gonflage lorsqu'il est relié à l'ouverture de gonflage, le ballonnet se remplit d'une solution saline.

9. Tige (1) selon l'une quelconque des revendications 1 à 6, laquelle comprend en outre des moyens d'adhérence/fixation (6) prévus dans la partie distale (3) et des moyens d'actionnement dans la partie proximale (2), les moyens d'adhérence/fixation (6) étant de tel façon que, lorsqu'ils sont appliqués dans l'urètre d'un patient et sous une action d'actionnement aux moyens d'actionnement, ils assurent l'adhérence à l'urètre d'un patient.

10. Tige selon la revendication précédente, dans laquelle les moyens d'adhérence/fixation (6) consistent en au moins trois bras articulés ou pièces de volume qui, dans une position non armée, sont disposés sur la surface de la tige (1) et, dans une position armée, achevée par moyen dudit actionnement, font saillie de la surface, et les moyens d'actionnement consistent en un élément configuré pour assurer l'actionnement desdits bras articulés et, de préférence :
• ledit élément configuré pour actionner lesdits bras articulés ou pièces de volume consiste en un bouton et un mécanisme de transmission relié entre le bouton et les bras articulés ou pièces de volume et est disposé à l'intérieur de la tige (1) de sorte que, sous l'actionnement du bouton, le mécanisme de transmission assure la saillie des bras articulés par rapport à la surface.

11. Tige (1) selon l'une quelconque des revendications 1 à 8, laquelle comprend en outre un bouton proximal (7) situé à l'extrémité de la partie proximale (2) et un mécanisme de transmission, et une section d'extrémité distale (8) comprenant les deux ouvertures est rotative, le bouton proximal (7) et le mécanisme de transmission étant disposés de telle façon que, sous l'effet de la rotation du bouton proximal (7), le mécanisme de transmission assure la rotation de la section d'extrémité distale (8) et, par conséquence, des deux ouvertures et du canal (4) respectif qui y est formé.

12. Tige (1) selon l'une quelconque des revendications précédentes, laquelle est faite d'un matériau métallique adapté à une application chirurgicale humaine, de préférence l'acier inoxydable ou le titane.

13. Tige (1) selon l'une quelconque des revendications 1 à 11, laquelle est faite d'un matériau polymère, de préférence un polyéther bloc amide, un polytétraméthylène glycol, un élastomère ou un polyéthylène.

14. Tige (1) selon l'une quelconque des revendications précédentes, dans laquelle la partie proximale (2) comprend des moyens de préhension, de préférence prévus dans l'extrémité de la partie proximale (2).

15. Kit de suture chirurgicale comprenant la tige (1) de l'une quelconque des revendications précédentes et une aiguille chirurgicale, cette aiguille chirurgicale ayant une courbure.
